# EUROPÄISCHE PATENTANMELDUNG

(11) **EP 1 762 217 A1**
(43) Veröffentlichungstag der Anmeldung: **14.03.2007**
(21) Anmeldenummer: 06120240.4
(22) Anmeldetag: 07.09.2006
(51) Int. Cl.: A61K 8/35, A61K 8/37, A61Q 17/00, A61Q 17/04

(54) **UV-Filterkomposition enthaltend PPG-3 Benzylethermyristat**

(30) Priorität: 09.09.2005 DE 102005044262
(71) Anmelder: Beiersdorf AG, 20245 Hamburg (DE)
(72) Erfinder: Lerg, Heike, 22303, Hamburg (DE); Mundt, Claudia, 28207, Bremen (DE); Steinforth, Melanie, 22303, Hamburg (DE)

(57) **Zusammenfassung**

Kosmetische Zubereitung für die topische Anwendung, dadurch gekennzeichnet, dass sie in einem kosmetisch akzeptablen Träger
a) mindestens einen UV-Filter gewählt aus der Gruppe 4-(tert.-Butyl)-4'-methoxydibenzoylmethan (CAS-Nr. 70356-09-1) und 4-Methoxyzimtsäure-2-ethylhexylester sowie
b) PPG-3 Benzylethermyristat (CAS 403517-45-3)

enthält.

## Beschreibung

Die vorliegende Erfindung betrifft eine kosmetische Zubereitung für die topische Anwendung, dadurch gekennzeichnet, dass sie in einem kosmetisch akzeptablen Träger
a) mindestens einen UV-Filter gewählt aus der Gruppe 4-(tert.-Butyl)-4'-methoxydibenzoylmethan (CAS-Nr. 70356-09-1) und 4-Methoxyzimtsäure-2-ethylhexylester sowie
b) PPG-3 Benzylethermyristat
enthält.

Der Trend weg von der vornehmen Blässe hin zur "gesunden, sportlich braunen Haut" ist seit Jahren ungebrochen. Um eine gebräunte Hautfarbe zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UV-B-Bereich (Wellenlänge: 280-320 nm) auf. Die übermäßige Einwirkung der UVB- und UV-A-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Kosmetische Sonnenschutzmittel sind hoch komplexe Substanzmischungen, deren häufig mangelnde Homogenität und Stabilität bei der Lagerung und Anwendung (UV-Stabilität) den Kosmetiker immer wieder vor Probleme stellt.

So ist aus der Familie der UV-A-Filtersubstanzen (im folgenden auch UV-A-Filter genannt) die Verbindungsklasse der Dibenzoylmethanderivate, insbesondere 4-(tert.-Butyl)-4'-methoxydibenzoylmethan als besonders leistungsstarke UV-Filter bekannt. Leider ist von dieser Verbindungsklasse ebenfalls bekannt, dass die Filter selbst relativ empfindlich gegenüber der UV-Strahlung sind und die Tendenz zeigen, sich unter der Einwirkung des UV-Lichtes mehr oder weniger schnell zu zersetzen. Die Photostabilität von Dibenzoylmethanderivaten ist also begrenzt und die UV-Lichtschutzfilterleistung nimmt bei der Anwendung der Filter auf der Haut im Verlaufe der Zeit ab.

Entsprechende Beobachtungen wurden auch bei dem UV-Filter 4-Methoxyzimtsäure-2-ethylhexylester gemacht.

Ein weiters Problem kosmetischer Zubereitungen stellt ihre mikrobiologische Stabilität dar. Kosmetische Zubereitungen, insbesondere kosmetische Sonnenschutzzubereitungen müssen über einen längeren Zeitraum (häufig über Monate und Jahre) im angebrochenen Zustand vor übermäßigem Befall durch Mikroorganismen geschützt werden, welche einzelne Bestandteile der Zubereitung verstoffwechseln und die Zubereitung insgesamt zersetzen können. Zum Schutz vor Mikroorganismen werden den Zubereitungen daher Konservierungsmittel zugesetzt, die jedoch u.a. aus Gründen des Schutzes vor Allergien, beim Verbraucher unbeliebt sind.

Es war daher die Aufgabe der vorliegenden Erfindung, die Mängel des Standes der Technik zu beseitigen und kosmetische Zubereitungen zu entwickeln, die für sich eine hohe Stabilität gegenüber der UV-Strahlung und Mikroorganismen aufweisen.

Insbesondere war es die Aufgabe der vorliegenden Erfindung eine kosmetische Zubereitung für die topische Anwendung auf der Haut, insbesondere zum Schutz vor den schädliche Einflüssen der UV-Strahlung, zu entwickeln, in denen Dibenzoylmethanderivate (insbesondere 4-(tert.-Butyl)-4'-methoxydibenzoylmethan) und/oder 4-Methoxyzimtsäure-2-ethylhexylester enthalten sind, deren photochemische Zersetzung gegenüber herkömmlichen Zubereitungen deutlich gebremst ist.

Überraschend gelöst werden die Aufgaben durch eine kosmetische Zubereitung für die topische Anwendung, dadurch gekennzeichnet, dass sie in einem kosmetisch akzeptablen Träger
a) mindestens einen UV-Filter gewählt aus der Gruppe 4-(tert.-Butyl)-4'-methoxydibenzoylmethan (CAS-Nr. 70356-09-1) und 4-Methoxyzimtsäure-2-ethylhexylester sowie
b) PPG-3 Benzylethermyristat (CAS 403517-45-3)
enthält.

Die erfindungsgemäße Zubereitung zeigt eine hervorragende Stabilität gegenüber UV-Strahlen. Auch zeigen die UV-Filter 4-(tert.-Butyl)-4'-methoxydibenzoylmethan und 4-Methoxyzimtsäure-2-ethylhexylester eine deutlich höhere Stabilität als in Zubereitungen ohne PPG-3 Benzylethermyristat. Die erfindungsgemäßen UV-Filter 4-(tert.-Butyl)-4'-methoxydibenzoylmethan und 4-Methoxyzimtsäure-2-ethylhexylester sind in der Zubereitung so stabil, dass auf den Einsatz des herkömmlichen Stabilisators p-Methylbenzylidencampher erfindungsgemäß vorteilhaft verzichtet werden kann.

Darüber hinaus weisen die erfindungsgemäßen Zubereitungen eine überraschend hohe UV-A-Balance (nach DIN 67502) und einen besonders langzeitstabilen SPF (Lichtschutzfaktor) auf. Die Zubereitungen haben nicht zuletzt ein besonders geringes Allergierisiko.

Erfindungsgemäß ist daher die Verwendung von PPG-3 Benzylethermyristat (CAS 403517-45-3) zur Erhöhung der UV-Stabilität photolabiler UV-Filter.

Es ist erfindungsgemäß vorteilhaft, wenn die Zubereitung PPG-3 Benzylethermyristat (CAS 403517-45-3) in einer Konzentration von 0,1 bis 10 Gewichts-%, und erfindungsgemäß bevorzugt, wenn die Zubereitung PPG-3 Benzylethermyristat in einer Konzentration von 0,5 bis 3 Gewichts-% enthält, wobei sich die Konzentrationsangaben auf das Gesamtgewicht der Zubereitung beziehen.

Erfindungsgemäß vorteilhaftes PPG-3 Benzylethermyristat (CAS 403517-45-3) kann beispielsweise unter dem Handelsnamen Crodamol STS bei der Firma Croda Chemicals erworben werden.

Erfindungsgemäß vorteilhafte Ausführungsformen der vorliegenden Erfindung sind dadurch gekennzeichnet, dass die Zubereitung den oder die UV-Filter gewählt aus der Gruppe 4-(tert.-Butyl)-4'-methoxydibenzoylmethan (CAS-Nr. 70356-09-1) und 4-Methoxyzimtsäure-2-ethylhexylester in einer Gesamtkonzentration von 2 bis 15 Gewichts-%, bezogen auf das Gesamtgewicht der Zubereitung, enthält.

Die Erfindung kann in den folgenden erfindungsgemäß bevorzugten Ausführungsformen vorliegen:
I) Kosmetische Zubereitung für die topische Anwendung, dadurch gekennzeichnet, dass sie in einem kosmetisch akzeptablen Träger
   a) 4-(tert.-Butyl)-4'-methoxydibenzoylmethan sowie
   b) PPG-3 Benzylethermyristat (CAS 403517-45-3)
   enthält.
   In dieser Ausführungsform ist es erfindungsgemäß vorteilhaft, wenn die Zubereitung 4-(tert.-Butyl)-4'-methoxydibenzoylmethan in einer Konzentration von 0,1 bis 10 Gewichts-% und bevorzugt in einer Konzentration von 1 bis 5 Gewichts-%, jeweils bezogen auf das Gesamtgewicht der Zubereitung enthält.
II) Kosmetische Zubereitung für die topische Anwendung, dadurch gekennzeichnet, dass sie in einem kosmetisch akzeptablen Träger
   a) 4-Methoxyzimtsäure-2-ethylhexylester sowie
   b) PPG-3 Benzylethermyristat (CAS 403517-45-3)
   enthält.
   In dieser Ausführungsform ist es erfindungsgemäß vorteilhaft, wenn die Zubereitung 4-Methoxyzimtsäure-2-ethylhexylester in einer Konzentration von 0,1 bis 10 Gewichts-% und bevorzugt in einer Konzentration von 3 bis 10 Gewichts-%, jeweils bezogen auf das Gesamtgewicht der Zubereitung enthält.
III) Kosmetische Zubereitung für die topische Anwendung, dadurch gekennzeichnet, dass sie in einem kosmetisch akzeptablen Träger
   a) 4-(tert.-Butyl)-4'-methoxydibenzoylmethan (CAS-Nr. 70356-09-1) und 4-Methoxyzimtsäure-2-ethylhexylester sowie
   b) PPG-3 Benzylethermyristat (CAS 403517-45-3)
   enthält.

In dieser Ausführungsform ist es erfindungsgemäß vorteilhaft, wenn die Zubereitung 4-(tert.-Butyl)-4'-methoxydibenzoylmethan in einer Konzentration von 0,1 bis 10 Gewichts-% und bevorzugt in einer Konzentration von 1 bis 5 Gewichts-%, jeweils bezogen auf das Gesamtgewicht der Zubereitung enthält.

Ferner ist es in dieser Ausführungsform erfindungsgemäß vorteilhaft, wenn die Zubereitung 4-Methoxyzimtsäure-2-ethylhexylester in einer Konzentration von 0,1 bis 10 Gewichts-% und bevorzugt in einer Konzentration von 3 bis 10 Gewichts-%, jeweils bezogen auf das Gesamtgewicht der Zubereitung enthält.

4-(tert.-Butyl)-4'-methoxydibenzoylmethan (CAS-Nr. 70356-09-1) kann bei der Firma Givaudan unter der Marke Parsol® 1789 und bei der Firma Merck unter der Handelsbezeichnung Eusolex® 9020 bezogen werden.

Methoxyzimtsäure-2-ethylhexylester, auch 2-Ethylhexyl-4-methoxycinnamat genannt, wird mit der INCI: Octyl Methoxycinnamat deklariert und ist bei der Firma DSM Nutritional Products Europe unter dem Handelsnamen Parsol MCX erhältlich.

Erfindungsgemäß vorteilhafte Ausführungsformen der vorliegenden Erfindung sind dadurch gekennzeichnet, dass die Zubereitung weitere organische UV-A- und/oder UV-B-Filter enthält.

So ist es erfindungsgemäß vorteilhaft, wenn die weiteren organischen UV-Filter gewählt werden aus der Gruppe der Verbindungen der Anthralinate, Cinnamate, Salicylate, Derivate des Camphers, Derivate des Benzophenons, Derivate der β, β-Diphenylacrylate, Derivate von Benzotriazol, Derivate von Benzalmalonaten, Derivate von Benzimidazolen, Imidazoline, Derivate von bis-Benzoazolyl, Derivate von p-Aminobenzoesäure, Derivate von Methylen-bis-(hydroxyphenylbenztriazol), polymere Filter, Silikonfilter, Dimere Derivate von α-Alkylstyren, 4, 4-Diarylbutadien. Hierzu zählen beispielsweise die folgenden UV-Filter, deren Namen in der für die Kosmetik üblichen INCI angegeben ist:
Derivate von p-Aminobenzoesäure
   - PABA
   - Ethyl PABA
   - Ethyl Dihydroxypropyl PABA
   - Ethylhexyl Dimetyl PABA
   - Glyceryl PABA
   - PEG-25 PABA
Salicylate
   - Homosalate
   - Ethylhexylsalicylat
   - Dipropylenglycolsalicylat
   TEA Salicylat
Cinnamate
   - Ethylhexyl Methoxycinnamate
   - Isopropyl Methoxy cinnamate
   - Isoamyl Methoxy cinnamate
   - Cinoxate
   - DEA Methoxycinnamate
   - Diisopropyl Methylcinnamate
   - Glyceryl Ethylhexaoate Dimethoxycinnamate
Derivate der β, β-Diphenylacrylate
   - Octocrylen
   - Etocrylen
Derivate des Benzophenons
   - Benzophenone-1
   - Benzophenone-2
   - Benzophenone-3
   - Benzophenone 4
   - Benzophenone 5
   - Benzophenone 6
   - Benzophenone 8
   - Benzophenone 9
   - Benzophenone 12
Derivate des Benzylidencamphers
   - 3-Benzylidencampher
   - Benzylidencamphersulfonsäure
   - Benzalkoniummethosulfatcampher
   - Terephthalidendicamphersulfonsäure
   - Polyacrylamidomethylbenzylidencampher
Derivate von Phenylbenzimidazol
   - Phenylbenzimidazolsulfonsäure
   - Dinatriumphenyldibenzimidazoltetrasulfonat
Derivate des Triazins
   - Anisotriazin (= Bis-Ethylhexyloxyphenol Methoxyphenyl Triazin)
   - Ethylhexyltriazon
   - Diethylhexylbutamidotriazon
   - 2,4,6-tris-(4'-aminodiisobutylbenzmalonat)-s-triazin
Derivate von Phenylbenzotriazol
   - Drometrizoltrisiloxan
   - Methylen bis-Benzotriazolyltetramethylbutylphenol
Anthralinate
   - Menthylanthralinat
Derivate von Imidazolinen
   - Ethylhexyldimethoxybenzylidendioxoimidazolinpropionat
Derivate des Benzalmalonats
   - Polyorganosiloxan mit Funktionen des Benzalmalonats
Derivate des 4.4-Diarylbutadiens
   - 1,1-dicarboxy(2,2'-dimethyl-propyl)-4,4-diphenylbutadien

Bei dieser Liste ist es erfindungsgemäß bevorzugt, wenn einer oder mehrere organische Filter gewählt werden aus der Gruppe der folgenden Verbindungen:
- Ethylhexylsalicylat,
- Octocrylen,
- Phenylbenzimidazolsulfonsäure,
- Terephtalidendicampfersulfonsäure,
- Benzophenon-3,
- Benzophenon-4,
- Benzophenon-5,
- 4-Methylbenzylidenkampfer,
- Dinatriumphenyldibenzimidaoltetrasulfonat,
- Anisotriazin (Ethylhexyloxyphenol Methoxyphenyl Triazin),
- Ethylhexyltriazon,
- Diethylhexylbutamidotriazon,
- 2,4,6-tris-(4'-aminodiisobutylbenzalmalonat)-s-triazin,
- Methylenbis-Benzotriazolyltetramethylbutylphenol,
- Drometrizoltrisiloxan,
- 1,1-Dicarboxy(2,2'-dimethylpropyl)-4,4-diphenylbutadien.
Erfindungsgemäß besonders bevorzugt ist es, wenn die Zubereitung einen oder mehrere UV-Filter gewählt aus der Gruppe der Verbindungen 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1 (dimethylpropyl)benzoxazol-2-yl-(4-phenyl)-imino]-6-(2-ethylhexyl)-imino-1,3,5-triazin; 4,4',4"-(1,3,5-Triazin-2,4,6-triyltriimino)-tris-benzoesäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; Ethylhexylsalicylat; Terephthalidendicamphersulfonsäure; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan / Dimethylsiloxan - Copolymer; Dimethicodiethylbenzalmalonat, enthält.

Auch ist es erfindungsgemäß vorteilhaft, wenn die erfindungsgemäße Zubereitung Pigmente und/oder Nanopigmente (auch Mikropigmente genannt, d.h. Pigmente mit einem durchschnittlichen Durchmesser von 5 bis 100 nm und bevorzugt zwischen 10 und 50 nm) von beschichteten und/oder unbeschichteten Metalloxiden enthält.

Derartige Pigmente und/oder Nanopigmente werden erfindungsgemäß vorteilhaft gewählt aus den Oxiden des Titans, Zinks, Eisens, Zirkoniums, Cers, und deren Mischungen.

Es ist erfindungsgemäß vorteilhaft, wen die erfindungsgemäße Zubereitung einen oder mehrere dieser weiteren UV-Filter enthält, wobei die Gesamtmenge der Filtersubstanzen 0,1 Gew.ichts-% bis 30 Gewichts-%, vorzugsweise 0,5 bis 10 Gewichts-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Es ist erfindungsgemäß vorteilhaft, wenn die erfindungsgemäße Zubereitung als weiteren Bestandteil mindestens eine Verbindung zur Bräunung der Haut enthält, beispielsweise Dihydroxyaceton (DHA).

Es ist erfindungsgemäß vorteilhaft, wenn die erfindungsgemäße Zubereitung als weitere Komponenten mindestens eine Verbindung gewählt aus der Gruppe der Verbindungen der Lipide, der organischen Lösungsmittel, der ionischen oder nichtionischen Verdickungsmittel, die Haut beruhigende Stoffe, Antioxidantien, Radikalfänger, Trübungsmittel, Stabilisatoren, Emulgatoren, Hauterweichungsmittel, Silikone, α-Hydroxysäuren, Entschäumer, Feuchthaltemittel, Vitamine, Substanz P Antagonisten, Füllstoffe, Polymere, Treibmittel, Säuren bzw. Basen zur pH-Wert-Einstellung, Farbstoffe.

Die Lipide können Öle, Wachse und deren Mischungen enthalten. Als Öl wird dabei eine bei Raumtemperatur flüssige lipophile Komponente aufgefasst. Als Wachse gelten erfindungsgemäß lipophile Komponenten, die bei Raumtemperatur fest sind und einen Schmelzpunkt von über 35 °C aufweisen. Zu den Lipiden zählen beispielsweise Fettsäuren, Fettalkohole oder Ester.

Zu den erfindungsgemäß vorteilhaften Ölen gehören beispielsweise Paraffinöle (Mineralöle), pflanzliche Öle (z.B. Macadamiaöl, Jojobaöl, Traubenkernöl, Weizenkeimöl), Squalan, Alkohole, Ester, Säuren, C12-C15 Alkylbenzoate, Octylpalmitat, Isopropyllanolat, Capryl-/Caprinsäure-Triglyceride, Ester und Ether von Oxyethylen und/oder Oxypropylen, Silikonöle (z.B. Cyclomethicon, Dimethicon, Polydimethylsiloxane).

Ferner gehören zu den erfindungsgemäß vorteilhaften Lipiden Vaseline, Paraffine, Carnaubawachs, hydriertes Rizinusöl, Bienenwachs, Myristylmyristat, Isopropylpalmitat.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist dabei erfindungsgemäß besonders bevorzugt, wenn die Zubereitung Tocopherylacetat enthält. Dabei ist es erfindungsgemäß bevorzugt, die Konzentration an Tocopherylacetat von 0,001 bis 10 Gew.-%, bezogen auf das Gesamtgewicht der Formulierung, zu wählen.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A.

Die erfindungsgemäße Zubereitung enthält in der Regel Konservierungsmittel. In einem solchen Fall ist es erfindungsgemäß vorteilhaft, wenn die Zubereitung Phenoxyethanol und/oder Parabene wie Methylparaben oder Propylparaben enthält. Diese Konservierungsmittel können erfindungsgemäß bevorzugt in einer Konzentration von 0,1 bis 2,0 Gewichts-%, bezogen auf das Gesamtgewicht der Zubereitung eingesetzt werden.

Es ist erfindungsgemäß vorteilhaft, wenn es sich bei der erfindungsgemäßen Zubereitung um eine Emulsion, Dispersion oder eine gelförmige Zubereitung handelt.

Die erfindungsgemäße Zubereitung kann dabei vorteilhaft in Form einer Creme, einer Milch, eines Gels, einer Gelcreme, eines Puders vorliegen.

Die Verwendung als Spray, Schaum, Tränkung für ein Tuch oder Stift ist erfindungsgemäß.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

In den folgenden Beispielen bedeutet:
- UVASorb® K2A = 2,4-bis-[5-1(dimethylpropyl)benzoxazol-2-yl-(4-phenyl)-imino]-6-(2-ethylhexyl)-imino-1,3,5-triazin [CAS Nr. 288254-16-0]
- Uvinul® A Plus = 2-(4'-Diethylamino-2'-hydoxybenzoyl)-benzoesäurehexylester (auch: Aminobenzophenon)

**1. O/W Emulsionen**

| | **1** | **2** | **3** | **4** | **5** | **6** | **7** |
|---|---|---|---|---|---|---|---|
| PEG-40 Castor Öl, Natrium Cetearyl Sulfat, Cetearyl Alkohol | | | | | | | 2,50 |
| Glycerinmonostearat (SE) | 1,00 | 2,00 | 3,00 | 1,00 | | 1,50 | 1,00 |
| Glyceryl Stearat Citrat | | | | | 2,00 | | |
| Stearinsäure | 3,00 | | 2,50 | 2,00 | | | |
| PEG-40 Stearat | | 2,00 | | | | 2,00 | |
| PEG-100 Stearat | | | 0,75 | | | | |
| Lauryl Methicon Copolyol | | | | 0,75 | | 0,50 | |
| Sorbitan Stearat | | | 0,75 | | | | |
| Cetyl Phosphat | | | 0,75 | | | | |
| Stearyl Alkohol | | | 3,00 | | 2,00 | 2,00 | |
| Cetyl Alkohol | 1,00 | 2,00 | | | 0,50 | | 1,50 |
| UVASorb® K2A | | | | 4,00 | | 5,00 | |
| Uvinul® A Plus | 2,50 | | | 0,25 | 1,00 | 0,50 | |
| Butylmethoxydibenzoylmethan | | 3,00 | | | | 2,00 | 4,50 |
| 4-Methylbenzylidencampher | | | 3,00 | | | | |
| Bis-Ethylhexyloxyphenol Methoxyphenyl Triazin | 1,00 | | | | | 1,00 | 3,00 |
| Dinatrium Phenyl Dibenzimidazol Tetrasulfonat | | | | 1,00 | | 2,00 | |
| Phenylbenzmidazol Sulfonsäure | | | | | | | 2,00 |
| Ethylhexyl Triazon | | | | 2,00 | | 2,00 | |
| Diethylhexyl Butamido Triazon | 2,00 | | | | | | |
| Ethylhexyl Methoxycinnamat | 3,50 | | 5,50 | 10,00 | 2,50 | | 9,50 |
| Octocrylen | | | | 5,00 | 9,00 | 7,50 | |
| Methylen Bis-Benztriazolyl Tetramethylbutylphenol | | | 2,00 | | 3,00 | | |
| Ethylhexylsalicylat | | 0,50 | 3,00 | | | | |
| Drometrizol Trisiloxan | | | 0,50 | | | 1,00 | |
| Terephtalidene Dicampher Sulfonsäure | | | | | | 2,00 | |
| Dimethylcodiethylbenzalmalonat | | | | | | 3,00 | |
| Titandioxid T 805 | 2,00 | | | | 1,00 | 0,50 | |
| Titandioxid MT-100Z | | | | 3,00 | 1,00 | | |
| C₁₂₋₁₅ Alkyl Benzoat | 1,00 | | | | | 2,00 | 5,50 |
| Octyldodecanol | 1,50 | | | 0,50 | | 4,00 | 5,50 |
| PPG-3 Benzylethermyristat | 0,80 | 1,50 | 2,50 | 4,50 | 10,00 | 7,50 | 1,00 |
| Dicaprylylether | | | 3,50 | | 2,00 | | |
| Paraffinöl | | 6,00 | | | | | |
| Butylenglycol Dicaprylat/ Dicaprat | | | | 5,00 | 3,00 | | |
| Cetearyl Isononanoat | 4,00 | | | | | 2,00 | |
| Dimethicon | 0,50 | 3,00 | 1,00 | | 2,00 | | |
| Cyclomethicon | | 3,00 | | 4,50 | | | |
| Dimethicon / Vinyl Dimethicon Crosspolymer | 4,00 | | | | | | |
| PVP Hexadecen Copolymer | | | | 0,50 | 1,00 | | |
| Glycerin | 7,50 | 10,00 | | 7,50 | 5,00 | | 1,00 |
| Xanthangummi | | 0,20 | 0,05 | | | | 0,40 |
| Butylenglycol | 5,00 | | | | | 7,00 | |
| Ascorbinsäure | | | | | | 3,50 | |
| Tocopherol | 0,20 | | | | | | |
| Taurin | 1,00 | | | | | | |
| Vitamin E Acetat | | 0,40 | 0,25 | 0,50 | 0,75 | | 0,10 |
| Retinol | | | | | 0,05 | | |
| Natrium Stärke Octenylsuccinat | | | | 0,20 | | 0,25 | 1,50 |
| Maisstärke | | 0,50 | | | | | 3,00 |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | 0,10 |
| Phenoxyethanol | 0,40 | 0,50 | | 0,40 | 0,50 | | 0,60 |
| EDTA | 0,20 | | 0,35 | 0,50 | 0,02 | | 0,20 |
| Ethanol | 2,00 | | 1,50 | | 3,00 | 5,00 | 8,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 6,0-7,5 | 4,5-7,0 | 6,5-8,5 | 5,0-7,0 | 6,0-8,0 | 4,0-6,0 | 5,0-7,5 |

**O/W Emulsionen**

| | **8** | **9** | **10** | **11** | **12** |
|---|---|---|---|---|---|
| Glycerinmonostearat (SE) | | | | 1,00 | |
| Glyceryl Stearat Citrat | 2,00 | 2,00 | 1,50 | | |
| Polyglyceryl-3- Methylglucose Distearat | | | | | 4,50 |
| Stearyl Alkohol | 2,00 | 2,00 | | | |
| Cetyl Alkohol | | | 2,00 | | 4,50 |
| Bis-Ethylhexyloxyphenol Methoxyphenyl Triazin | 2,00 | 2,00 | 1,50 | | |
| Diethylhexyl Butamido Triazon | | 1,00 | 2,00 | | |
| Ethylhexyl Methoxycinnamat | 2,00 | 6,00 | 5,00 | 9,50 | 2,00 |
| Butylmethoxydibenzoylmethan | | | | 3,50 | 2,00 |
| Octocrylen | 2,00 | 9,00 | | | |
| Titandioxid T 805 | | 3,00 | 2,00 | | |
| C12-15 Alkyl Benzoat | | | 3,00 | 3,00 | 1,00 |
| Crodamol STS | 1,00 | 0,10 | | 2,00 | 3,00 |
| Hydriertes Kokosfettsäureglycerid | 1,00 | 1,00 | 3,00 | | |
| Dicaprylylether | 5,00 | 2,00 | 6,00 | | |
| Octyldodecanol | 6,00 | 5,00 | 4,00 | 1,00 | 2,00 |
| Butylenglycol Dicaprylat/ Dicaprat | 2,00 | 2,00 | | | |
| Caprylsäure/Caprinsäuretriglyc erid | | | | 2,00 | 1,50 |
| Dimethicon | | | 2,00 | | |
| Cyclomethicon | 2,00 | 1,00 | | 3,00 | |
| Sorbitol | | | | 2,50 | |
| Acrylat/C10-30 Alkyl Acrylat Crosspolymer | 0,10 | 0,10 | 0,05 | | |
| PVP Hexadecen Copolymer | 0,50 | 0,50 | | | |
| Glycerin | 8,00 | 6,00 | 5,00 | 3,00 | 3,00 |
| Xanthangummi | 0,40 | 0,40 | 0,25 | 0,30 | 0,10 |
| Butylenglycol | | | 3,00 | 3,00 | |
| Linolsäure | 1,00 | | | | |
| Nachtkerzenöl | | 1,00 | | | |
| Genistein | 0,05 | | | | |
| Silymarin | | 0,1 | | | |
| Phosphatidylcholin | | | | 0,06 | |
| Silybin | | | | 0,03 | |
| Carnitin | | | 0,5 | | |
| Vitamin E Acetat | 0,50 | | 0,30 | 0,40 | 0,40 |
| Dihydroxyaceton | | | | 5,00 | 3,00 |
| DMDM Hydantoin | 0,60 | 0,60 | 0,50 | | |
| Methylparaben | | | | 0,30 | 0,30 |
| Phenoxyethanol | 0,40 | 0,40 | 0,35 | 0,50 | 0,50 |
| EDTA | 1,00 | 1,00 | 1,00 | | 1,00 |
| Ethanol | | | 3,00 | 3,00 | |
| Insekt Repellent 3535 | | | | 4,00 | 5,00 |
| Parfüm | 0,40 | 0,40 | 0,40 | 0,40 | 0,40 |
| Wasser | ad 100 | ad 100 | ad 100 | ad 100 | ad 100 |
| Neutralisationsmittel (Natriumhydroxid, Kaliumhydroxid) | qs | qs | qs | qs | qs |
| pH-Wert | 5,0-7,0 | 5,0-7,0 | 5,0-7,0 | 5,0-7,0 | 5,0-7,0 |

**O/W-Emulsionen**

| | **13** | **14** | **15** | **16** | **17** | **18** | **19** |
|---|---|---|---|---|---|---|---|
| Glyceryl Stearat, Ceteareth-12, Ceteareth-20, Cetearyl Alcohol, Cetyl Palmitat | | | | | | | 1,50 |
| Glycerinmonostearat (SE) | | 4,00 | | | | | |
| Glyceryl Stearat Citrat | 2,00 | | | | | | |
| Polyglyceryl-3- Methylglucose Distearat | | | | | 2,00 | | |
| Cetearyl Glucosid & Cetearyl Alkohol | | | | | | 2,00 | |
| Triceteareth-4 Phosphat | | | 1,20 | | | | |
| Trilaureth-4 Phosphat | | | | 2,00 | | | |
| Ceteareth-6 | | | 0,50 | 0,50 | | | |
| Sorbitan Stearat | | | 0,75 | 1,00 | 1,00 | | |
| Cetyl Phosphat | | | | | 2,00 | | |
| Stearyl Alkohol | | 2,50 | 3,00 | | | | 1,50 |
| Cetyl Alkohol | 2,50 | 1,00 | | | 0,50 | 2,00 | 2,00 |
| Phenethyl Benzoat | 1,50 | | | 0,50 | | 4,00 | 10,00 |
| PPG-3 Benzylethermyristat | 2,00 | 1,00 | 1,50 | 0,50 | 0,75 | 0,90 | 5,00 |
| UVASorb® K2A | 1,00 | | | 4,00 | | 5,00 | |
| Uvinul® A Plus | 3,00 | | 0,50 | 0,25 | 1,00 | 0,50 | |
| Butylmethoxydibenzoylmethan | | 4,50 | | | 1,50 | | 4,50 |
| Bis-Ethylhexyloxyphenol Methoxyphenyl Triazin | | | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | 3,00 | | |
| Ethylhexyl Triazon | 2,00 | 1,50 | | 2,00 | | 2,00 | |
| Diethylhexyl Butamido Triazon | | | | | | | |
| Ethylhexyl Methoxycinnamat | 5,00 | | 1,00 | 10,00 | 2,00 | 4,50 | |
| Octocrylen | | 4,00 | | 5,00 | 9,00 | 7,50 | 2,50 |
| Methylen Bis-Benztriazolyl Tetramethylbutylphenol | | | 2,00 | | 3,00 | | |
| Ethylhexylsalicylat | | | | | | | 5,00 |
| Drometrizol Trisiloxan | | 1,00 | 0,50 | | | 1,00 | |
| Terephtalidene Dicampher Sulfonsäure | | 1,00 | | | | | |
| Dimethylcodiethylbenzalmalonat | | | 5,00 | | | | |
| Titandioxid MT-100Z | 1,00 | | | 3,00 | 1,00 | | |
| Zinkoxid Z-Cote HP1 | | | | | | 3,00 | |
| Corapan TQ® | | | | | | | 6,00 |
| C₁₂₋₁₅ Alkyl Benzoat | | 2,50 | | | | 7,00 | 5,00 |
| Dicaprylylether | | | 3,50 | | 2,00 | | |
| Butylenglykol Dicaprylat/ Dicaprat | 5,00 | | | 5,00 | 3,00 | | |
| Cetearyllsononanoat | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Süßholzextrakt | | | | | | 0,50 | |
| Licochalcon | | | 0,05 | | | | |
| Curcumin | 0,05 | | | | | | |
| beta-Carotin | | | | 0,10 | | | |
| Ceramid III | | | | | 0,30 | | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Alkohol | | 2,00 | 1,50 | | 3,00 | 5,00 | 1,00 |
| Insekt Repellent 3535 | | | 5,00 | | | | |
| Meersalz | 0,10 | | | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (NaOH, KOH) | qs | qs | qs | qs | qs | qs | qs |
| pH-Wert | 4,5-6,0 | 4,5-7,0 | 5,5-7,5 | 5,0-7,0 | 5,5-7,5 | 4,0-7,0 | 4,0-7,5 |

**O/W Emulsionen**

| | **20** | **21** | **22** | **23** | **24** | **25** | **26** |
|---|---|---|---|---|---|---|---|
| PEG-40 Castor Öl, Natrium Cetearyl Sulfat, Cetearyl Alkohol | 2,50 | 3,50 | 2,50 | 2,25 | 2,00 | 2,50 | 2,50 |
| Glycerinmonostearat (SE) | 1,00 | 1,50 | 1,00 | 1,25 | 1,50 | 1,00 | 1,00 |
| Cetyl Alkohol | 1,50 | 1,00 | 1,50 | 2,00 | 1,00 | | |
| Uvinul® A Plus | | | | | 4,00 | | |
| Butylmethoxydibenzoylmethan | 4,90 | 4,50 | 2,00 | 3,50 | 2,50 | 4,50 | 4,50 |
| Bis-Ethylhexyloxyphenol Methoxyphenyl Triazin | 1,50 | 3,00 | 2,00 | 2,00 | | 3,00 | 3,00 |
| Phenylbenzmidazol Sulfonsäure | | 2,00 | 2,00 | | | 2,00 | 2,00 |
| Ethylhexyl Triazon | | 1,50 | | 3,00 | | | |
| Diethylhexyl Butamido Triazon | 1,00 | | 0,50 | 1,00 | 1,50 | | |
| Ethylhexyl Methoxycinnamat | 4,50 | | 3,00 | 4,50 | | 9,50 | 9,50 |
| Octocrylen | 3,00 | | | 1,00 | | | |
| Ethylhexylsalicylat | | | | 4,50 | 3,50 | | |
| Titandioxid T 805 | | | | | | 1,00 | |
| C₁₂₋₁₅ Alkyl Benzoat | 5,50 | 5,50 | 3,50 | 5,50 | 4,50 | 5,50 | 5,50 |
| Octyldodecanol | 5,50 | 5,50 | 7,50 | 5,50 | 4,50 | 5,50 | 5,50 |
| Acrylat/C10-30 Alkyl Acrylat Crosspolymer | | | | | | 0,20 | 0,20 |
| Myristyl Myristat | | | | | | 1,00 | 1,50 |
| Risocast DA-H | 0,25 | 0,50 | 1,00 | 1,50 | 0,30 | 1,00 | 0,50 |
| PPG-3 Benzylethermyristat | 1,25 | 1,75 | 1,00 | 0,50 | 1,50 | 1,00 | 1,00 |
| Paraffinöl | | 2,00 | | | | | |
| Butylenglycol Dicaprylat/ Dicaprat | 1,00 | | | | | | |
| Cetearyl Isononanoat | | | | | 2,00 | | |
| Dimethicon | | | 2,00 | | | | |
| Cyclomethicon | | | 5,00 | 3,00 | | | |
| Dimethicon / Vinyl Dimethicon Crosspolymer | | | | | 0,30 | | |
| PVP Hexadecen Copolymer | | 0,50 | | 0,50 | | | |
| Glycerin | 7,50 | 1,00 | 2,00 | 1,00 | 1,00 | 1,00 | 1,00 |
| Xanthangummi | | | | | | 0,40 | 0,40 |
| Methylpropandiol | | | 2,00 | | 1,00 | | |
| Vitamin E Acetat | 2,00 | 0,10 | 0,50 | 0,10 | | | |
| Natrium Stärke Octenylsuccinat | 1,50 | 1,50 | 1,50 | | 1,50 | 1,50 | 1,50 |
| Maisstärke | 2,00 | 3,00 | | 1,00 | 3,00 | 3,00 | 3,00 |
| Dihydroxyaceton | | | 2,00 | | | | |
| Methylparaben | 0,10 | 0,10 | 0,20 | | 0,10 | 0,10 | 0,10 |
| Phenoxyethanol | 0,60 | 0,50 | 0,60 | 0,60 | 0,60 | 0,60 | 0,60 |
| EDTA | 0,20 | 0,20 | 0,20 | 0,20 | 0,20 | 0,20 | 0,20 |
| Ethanol | 8,00 | 8,00 | 8,00 | 8,00 | 8,00 | 8,00 | 8,00 |
| Insekt Repellent 3535 | | | | 0,50 | | | |
| Parfüm | 0,40 | 0,40 | 0,40 | 0,40 | 0,40 | 0,40 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 5,0-7,5 | 5,0-7,5 | 5,0-7,5 | 5,0-7,5 | 5,0-7,5 | 5,0-7,5 | 5,0-7,5 |

**2. Schaumförmige O/W- Emulsionen:**

| | **1** | | **2** | |
|---|---|---|---|---|
| | Gew.-% | Vol.-% | Gew.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglycerid | 4,00 | | 2,00 | |
| C12-15 Alkyl Benzoat | 7,00 | | 8,50 | |
| PPG-3 Benzylethermyristat | 0,50 | | 1,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Butylmethoxydibenzoylmethan | | | 2,00 | |
| Terephthaliden Dicamphersulfonsäure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| Na₂H₂EDTA | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| Emulsion 1 | | 70 | | |
| Emulsion 2 | | | | 35 |
| Gas (Stickstoff, Sauerstoff oder Kohlendioxid) | | 30 | | |
| Gas (Luft) | | | | 65 |

**weitere Schaumförmige O/W- Emulsionen:**

| | **3** | **4** | **5** |
|---|---|---|---|
| Stearinsäure | 2,00 | 2,00 | |
| Palmitinsäure | | | 1,50 |
| Cetylalkohol | 2,50 | 2,00 | |
| Stearylalkohol | | | 3,00 |
| PEG-100 Stearat | | | 3,50 |
| PEG-40 Stearat | | 2,00 | |
| PEG-20-Stearat | 3,00 | | |
| Sorbitanstearat | | 0,80 | |
| C12-15 Alkyl Benzoat | 5,00 | | |
| C12-13 Alkyl Tartrat | | | 4,00 |
| PPG-3 Benzylethermyristat | 2,00 | 1,00 | 0,50 |
| Butylenglycol Dicaprylat/Dicaprat | | 6,00 | |
| Dicaprylyl Ether | | | 2,00 |
| Cyclomethicon | | 2,00 | 3,00 |
| Butylenglycol | 1,00 | | |
| Isohexadecan | 2,00 | | |
| Propylenglykol | | | 5,00 |
| Glycerin | 5,00 | 7,00 | |
| Butylmethoxydibenzoylmethan | 1,00 | 4,50 | 2,00 |
| Uvinul A Plus® | 2,00 | | |
| Ethylhexyltriazon | 2,00 | 2,00 | 2,00 |
| Octocrylen | 2,00 | | |
| Bis-Ethylhexyloxyphenol Methoxyphenyltriazin | | 3,00 | 3,00 |
| Vitamin E Acetat | | 0,5 | |
| BHT | | | 0,10 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

**weitere Schaumförmige O/W- Emulsionen:**

| | **6** | **7** | **8** | **9** |
|---|---|---|---|---|
| Stearinsäure | 1,50 | | | |
| Palmitinsäure | | | 3,00 | 3,00 |
| Cetylalkohol | | 3,00 | | |
| Cetylstearylalkohol | | | 2,00 | 2,00 |
| Stearylalkohol | 3,00 | | | |
| PEG-100 Stearat | | 4,00 | | |
| PEG-40 Stearat | 3,00 | | | |
| PEG-20-Stearat | | | 3,00 | 3,00 |
| Sorbitanstearat | 1,00 | | | |
| Tridecyl Trimellitate | | 5,00 | | |
| C12-15 Alkyl Benzoat | | | 3,00 | 3,00 |
| PPG-3 Benzylethermyristat | 2,00 | 1,00 | 0,10 | 6,00 |
| Butylenglycol Dicaprylat/Dicaprat | 8,00 | | | |
| Octyldodecanol | | 2,00 | | |
| Kokosfettsäureglycerid | | | | 2,00 |
| Dicaprylyl Ether | | | 2,00 | 2,00 |
| Cyclomethicon | | | | |
| Dimethicon | 1,00 | | 2,00 | 2,00 |
| Isohexadecan | | 3,00 | | |
| Methylpropandiol | | 4,00 | | |
| Glycerin | 5,00 | | 6,00 | 6,00 |
| NeoHeliopan® AP | | 2,00 | | |
| Phenylbenzimidazol Sulfonsäure | 1,00 | 4,00 | 1,00 | 1,00 |
| Ethylhexylmethoxycinnamat | 5,00 | 7,50 | 4,00 | 4,00 |
| Diethylhexylbutamidotriazon | 1,00 | | | |
| Butyl Methoxydibenzoylmethan | 2,50 | | 2,00 | 2,00 |
| Bis-Ethylhexyloxyphenol Methoxyphenyltriazin | 2,00 | | | |
| Vitamin E Acetat | 0,20 | | 0,30 | 0,30 |
| BHT | | 0,05 | | |
| Na2H2EDTA | | | 0,40 | 0,40 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Kaliumhydroxid | | | | q.s. |
| Wasser | ad 100,0 | ad 100,0 | ad 100,0 | ad 100,0 |

Zur Herstellung des Schaums werden 80-97 Vol.-% der Emulsion I mit 3-20 Vol.-% eines geeigneten Gases (z.B. Propan/ Butan, Druckluft, Stickstoff) aufgeschäumt.

**3. Dünnflüssige bis sprühbare W/O-Emulsionen**

| | **1** | **2** | **3** | **4** | **5** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | | 2,00 | 0,50 | 4,00 | 0,25 |
| Butylmethoxydibenzoylmethan | | | 3,00 | 1,50 | |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| 4-Methylbenzylidencampher | 4,00 | | | | |
| Methylen Bis-Benztriazolyl Tetramethylbutylphenol | | | | | 3,00 |
| Ethylhexyl Methoxycinnamat | 3,00 | 4,00 | 2,50 | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 5,00 | | | |
| C12-15 Alkyl Benzoat | 7,00 | | 2,00 | | |
| Mineral Öl | 3,00 | | | | 2,00 |
| PPG-3 Benzylethermyristat | 2,00 | 5,00 | 6,00 | 0,50 | 1,00 |
| Kokosfettsäureglycerid | | 2,00 | | 5,00 | |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Alkohol | 2,00 | | | 5,00 | 1,00 |
| Farbstoff, öllöslich | 0,02 | | | | |
| Parfüm | 0,30 | 0,45 | 0,35 | | 0,15 |
| Wassser | ad. 100 | ad. 100 | ad. 100 | ad. 100 | ad. 100 |

**4. W/O Emulsionen (Cremes & Lotions)**

| | **1** | **2** | **3** | **4** | **5** | **6** |
|---|---|---|---|---|---|---|
| Cetyldimethicon Copolyol | | | | | 4,00 | |
| Polyglyceryl-2-dipolyhydroxystearat | 5,00 | 4,50 | | | | 4,50 |
| Polyglyceryl-3-Diisostearat | 1,75 | 1,50 | | | | |
| PEG-30-dipolyhydroxystearat | | | 3,00 | 5,00 | | |
| Bis-Ethylhexyloxyphenol Methoxyphenyl Triazin | | | 3,00 | 1,50 | | |
| Butyl Methoxydibenzoylmethan | 0,50 | | 4,00 | | | |
| UVASorb® K2A | | | | 2,00 | 2,50 | |
| Uvinul® A Plus | 3,00 | 1,00 | | 3,00 | 0,25 | 2,50 |
| Phenylbenzmidazol Sulfonsäure | | 4,00 | | | 2,00 | 0,50 |
| Ethylhexyl Methoxycinnamat | 0,50 | 8,00 | 2,50 | 9,50 | 5,00 | 4,00 |
| Diethylhexyl Butamido Triazon | 3,00 | 1,00 | | 1,00 | | 3,00 |
| Ethylhexyl Triazon | | | 4,00 | 2,00 | 4,00 | |
| Octocrylen | 7,00 | | | | | 2,50 |
| Titandioxid Uvinul® T 805 | 2,00 | 1,00 | 5,00 | | | |
| Titandioxid MT-100 TV | | | | | | 2,00 |
| Zinkoxid Z-Cote® HP1 | 2,50 | | | 3,00 | | |
| Corapan TQ® | | | 6,00 | | | |
| Mineralöl | | | 6,00 | 5,00 | | |
| Kokosfettsäureglycerid | 4,00 | 6,50 | | 5,00 | | |
| C12-15 Alkyl Benzoat | | | 2,00 | | 9,00 | |
| PPG-3 Benzylethermyristat | 4,00 | 1,00 | 7,00 | 8,00 | 1,00 | 2,00 |
| Dicaprylylether | 10,00 | | | | | 7,00 |
| Butylenglycol Dicaprylat/Dicaprat | | | 9,00 | 7,00 | 8,00 | 4,00 |
| Cyclomethicon | 2,00 | | | | | 2,00 |
| PVP Eicosene Copolymer | 0,50 | | | | 1,50 | 1,00 |
| Trinatrium EDTA | 1,00 | | 1,00 | 0,50 | 0,35 | |
| Ethylhexyloxyglycerin | | 0,30 | | | | 0,50 |
| Methylpropandiol | | | | | | 7,50 |
| Glycerin | 5,00 | 7,50 | 6,00 | 8,00 | 7,50 | 2,50 |
| Butylenglykol | | 2,50 | | | | |
| Glycin Soja | | 1,00 | | | | |
| MgSO₄ | 1,00 | 0,50 | 0,30 | 0,30 | 0,50 | |
| Milchsäure & Natriumsalz der Milchsäure | 1,00 | 0,50 | | | | 0,85 |
| Vitamin E | 0,50 | | 0,50 | 1,00 | | 1,00 |
| DMDM Hydantoin | | 0,60 | | | 0,20 | |
| Methylparaben | 0,50 | | | | 0,15 | |
| Phenoxyethanol | 0,50 | 0,40 | 0,50 | 0,60 | 1,00 | 0,60 |
| Dihydroxyaceton | | | | | 5,50 | |
| Alcohol | 3,00 | | 2,00 | 3,00 | | 1,00 |
| Parfüm | 0,20 | | 0,20 | 0,20 | | 0,20 |
| Wasser | ad 100 | ad 100 | ad 100 | ad 100 | ad 100 | ad 100 |

**5. Hydrodispersionen (zur Verwendung als Lotion oder Spray)**

| | **1** | **2** | **3** | **4** | **5** | **6** |
|---|---|---|---|---|---|---|
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| UVASorb® K2A | | | | | 3,50 | |
| Uvinul® A Plus | 0,25 | | | 0,50 | 2,00 | 1,50 |
| Butyl Methoxydibenzoylmethan | 0,75 | 4,50 | 3,50 | 1,00 | | |
| Bis-Ethylhexyloxyphenol Methoxyphenyl Triazin | | 2,00 | | 0,25 | | |
| Terephthaliden Dicampher Sulfonsäure | | | | | | 0,50 |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | 5,50 | 5,00 | 8,00 |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Titandioxid MT-100 Z | 0,50 | 2,00 | 1,00 | 2,00 | 3,00 | 1,00 |
| C12-15 Alkyl Benzoat | 2,00 | | 2,50 | | | |
| PPG-3 Benzylethermyristat | 1,00 | 0,50 | 0,20 | 1,50 | 2,50 | 5,00 |
| C18-36 Triglycerid Fettsäure | | | 1,00 | | | |
| Butylenglycol Dicaprylat/Dicaprat | | | | | 6,00 | |
| Dicaprylyl Carbonat | | 3,00 | | | | |
| Dicaprylylether | | 2,00 | | | | |
| Cyclomethicon | | | | 1,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α-Glycosylrutin | | | | | 0,25 | |
| Trinatrium EDTA | | 1,00 | 1,00 | 0,10 | 0,20 | |
| lodopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 4,5-5,5 | 5,0-7,0 | 5,0-7,0 | 5,0-7,0 | 4,0-6,0 | 5,0-7,5 |

**6. Gelcremes**

| | **1** | **2** | **3** | **4** | **5** |
|---|---|---|---|---|---|
| Carbomer | 0,125 | 0,125 | 0,125 | 0,125 | 0,125 |
| Shea Butter | | | | 1,00 | |
| Mineralöl | | | | | 6,00 |
| Octyldodecanol | | | | 1,50 | |
| Caprylsäure/Caprinsäuretriglycerid | | | 4,00 | | |
| Dicaprylyl Carbonat | | 9,00 | | 1,00 | 3,00 |
| X-Tend 226 | 1,00 | | 3,00 | | 2,50 |
| PPG-3 Benzylethermyristat | 0,50 | 1,00 | 2,50 | 6,00 | 0,75 |
| Butylmethoxydibenzoylmethan | 2,00 | 4,50 | 0,50 | 3,50 | 4,50 |
| Ethylhexyl Methoxycinnamat | 9,50 | 0,50 | 7,00 | 5,50 | 5,00 |
| Dimethicon | | | | 0,50 | |
| Cyclomethicon | 9,00 | 2,00 | 3,00 | 2,00 | 1,00 |
| Diazolidinylharnstoff | 0,20 | 0,20 | 0,20 | 0,20 | 0,20 |
| Phenoxyethanol + Ethyl-, Methyl-, Propyl- Butyl-, Isobutylparaben | 0,50 | 0,50 | 0,50 | 0,50 | 0,50 |
| Parfum | | | | 0,25 | 0,25 |
| Glycerylstearatcitrat | 1,00 | 1,00 | 1,00 | 1,00 | 1,00 |
| Hydrierte Kokosfettsäureglycerid | 1,00 | 1,00 | 1,00 | 1,00 | 1,00 |
| Ammonium Acryloyldimethyltaurat/VP Copolymer | | | | | 0,125 |
| Hydroxyethylcellulose | 0,375 | 0,375 | 0,375 | 0,375 | 0,375 |
| Menthol | 0,10 | 0,50 | 1,00 | 0,10 | 0,10 |
| Wasser + Alcohol Denat | | | | 3,00 | |
| Wasser + Blue 1 | | 0,200 | 0,60 | 0,40 | |
| Wasser + Glycerin | 10,00 | 10,00 | 10,00 | 10,00 | 10,00 |
| Xanthangummi | 0,125 | 0,125 | 0,125 | 0,125 | |
| Wasser | ad 100,0 | ad 100,0 | ad 100,0 | ad 100,0 | ad 100,0 |
| Neutralisationsmittel (Natriumhydroxid, Kaliumhydroxid) | qs | qs | qs | qs | qs |
| pH-Wert | 4,5-5,5 | 6,5-8,5 | 5,0-7,0 | 4,0-6,0 | 5,0-7,5 |

**7. Ölgele**

| | **1** | **2** | **3** | **4** | **5** |
|---|---|---|---|---|---|
| Octyldodecanol | 9,00 | 9,00 | | | |
| Caprylsäure/Caprinsäuretriglycerid | 1,00 | | 1,00 | | |
| C12-15- Alkyl Benzoate | | | | 5,00 | 4,00 |
| Butylenglycol Dicaprylat/Dicaprat | | 9,00 | | | 3,00 |
| PPG-3 Benzylethermyristat | 2,00 | 1,00 | 1,00 | 0,25 | 5,00 |
| Dicaprylylether | 9,00 | | | 4,00 | |
| Dicaprylylcarbonat | | 7,00 | | | |
| Ethyl Galaktomannan (N-Hance® AG 200) | 3,50 | | | | 4,00 |
| C20-40 Fettsäuren + Polyethylen (Performacid®350) | | | | 3,60 | |
| Hydroxyoctacosanyl Hydroxystearat | 2,00 | | | | |
| Disteardimonium Hectorit | 1,00 | | | | 1,00 |
| Cetyl Dimethicon | 0,50 | | 4,50 | | |
| Cyclomethicon | | | 15,00 | | 5,00 |
| UVASorb® K2A | | | | | 1,00 |
| Uvinul® A Plus | 1,00 | 3,50 | 2,75 | 2,00 | 0,50 |
| Ethylhexylmethoxycinnamat | 6,00 | 4,25 | 8,40 | 10,00 | 3,00 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,00 |
| Polysaccharid-N-alkylurethanen, Inulincarbamat | 1,50 | 4,50 | 5,50 | 1,00 | 3,00 |
| Phenoxyethanol | 0,50 | | | | |
| Parfüm, Farbstoff | q.s. | q.s. | q.s. | q.s. | q.s. |
| Mineralöl | ad. 100 | ad. 100 | ad. 100 | | |
| Reisöl | | | | ad. 100 | ad. 100 |

**8. Feststoffstablisierte Emulsionen**

| | **1** | **2** | **3** | **4** | **5** |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 4,00 | 6,00 | | |
| C12-15- Alkyl Benzoat | | | | | 3,00 |
| Butylene Glykol Dicaprylat/Dicaprat | | | | | 3,00 |
| PPG-3 Benzylethermyristat | 2,00 | 1,00 | 1,00 | 2,50 | 0.50 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinöl | | | 2,50 | | 5,00 |
| Hydroxypropyl Methylcellulose | 0,15 | | | | 0,05 |
| Dimethicon | | | 4,50 | | |
| UVASorb® K2A | 2,00 | 5,00 | 3,00 | 1,50 | 1,00 |
| Bis-Ethylhexyloxyphenol Methoxyphenyl Triazin | 1,00 | 3,00 | 0,75 | 1,00 | 1,00 |
| Terephthaliden Dicampher Sulfonsäure | | 2,00 | | | 0,50 |
| Phenylbenzmidazol Sulfonsäure | 2,00 | 0,50 | 1,00 | | |
| Butylmethoxydibenzoylmethan | | 0,50 | 2,75 | 2,00 | 0,50 |
| Ethylhexylmethoxycinnamat | 6,00 | 1,50 | | 2,00 | 3,00 |
| Octocrylen | 3,50 | | 7,50 | | |
| Ethylhexyl Salicylat | | 3,50 | | | 4,00 |
| Diethylhexyl Butamido Triazon | | | | | 4,00 |
| Titandioxid Eusolex® T-2000 | | 2,00 | 4,00 | 2,00 | 4,00 |
| Titandioxid T 805® | | | | | 3,00 |
| Silica Dimethyl Silylat | | | 1,00 | | |
| Bornitrid | 2,00 | | | 3,00 | |
| Tapioca Stärke | | | | 1,00 | |
| Natriumchlorid | 1,00 | 1,00 | 1,00 | 1,00 | |
| Glycerin | 5,00 | 10,00 | | 6,00 | 10,00 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alkohol | | 5,00 | | 2,50 | |
| Parfüm | 0,45 | 0,20 | | | 0,45 |
| Wasser | ad 100 | ad 100 | ad 100 | ad 100 | ad 100 |

### 9. Gele:

### Beispiel 1 (Eye Shadow Gel):

| | Gew.-% |
|---|---|
| PEG-8 (Polyethylenglycol 400) | 2,00 |
| Ethanol | 5,00 |
| Aristoflex AVC | 1,50 |
| PPG-3 Benzylethermyristat | 1,00 |
| Butylmethoxydibenzoylmethan | 0,50 |
| Glycerin | 2,00 |
| Panthenol | 0,50 |
| Tocopherolacetat | 0,50 |
| Timiron Splendid blue® (Merck KgaA) | 4,50 |
| Chromoxid grün | 1,00 |
| Parfüm, Konservierungsmittel, NaOH, Komplexbildner, Farbstoffe, Antioxidantien etc. | q.s. |
| Wasser | ad 100,00 |

### Beispiel 2 (Hiqhliqhter Gel):

| | Gew. % |
|---|---|
| Carbomer | 1,50 |
| Glycerin | 2,50 |
| 1,3 Butylenglycol | 1,50 |
| PPG-3 Benzylethermyristat | 1,50 |
| Butylmethoxydibenzoylmethan | 2,00 |
| Glitzerpigmente (z.B. Helicone HC Scarabeus, Wacker) | 1,00 |
| EDTA | 0,20 |
| Dimethicone | 1,50 |
| Parfüm, Konservierungsmittel, NaOH, Farbstoffe, Antioxidantien, etc. | q.s. |
| Wasser | ad 100,00 |

### Beispiel 3 (Eye Liner Gel):

| | Gew. % |
|---|---|
| Perlglanzpigmente | 10,00 |
| Eisenoxid | 3,00 |
| Silica | 2,00 |
| Aristoflex AVC | 1,00 |
| Hyroxyproylethyl Cellulose | 0,35 |
| Citric Acid | q.s. |
| Glycerin | 5,00 |
| PPG-3 Benzylethermyristat | 0,50 |
| Octyl Methoxycinnamat | 2,00 |
| Butyl Methoxydibenzoylmethan | 1,00 |
| PVP/VA Copolymer | 2,00 |
| Parfüm, Konservierungsmittel, Farbstoffe, NaOH, Komplexbildner, Antioxidantien, etc. | q.s. |
| Wasser | ad 100,00 |

### 10. Make-up

### Beispiel 1 (Emulsions-Make-up):

| | Gew. % |
|---|---|
| PEG-30 Stearat | 2,00 |
| Glycerinmonostearat | 1,00 |
| Stearinsäure | 1,00 |
| Cyclomethicon | 7,00 |
| Octyldodecanol | 4,00 |
| PPG-3 Benzylethermyristat | 2,00 |
| Isopropyl Lanolat | 4,00 |
| Squalan | 2,00 |
| Octyl Methoxycinnamat | 2,00 |
| Butyl Methoxydibenzoylmethan | 1,00 |
| Xanthangummi | 0,20 |
| Glycerin | 5,00 |
| Butylenglkcol | 2,00 |
| Vitamin E Acetat | 1,00 |
| Magnesiumsilikat | 1,00 |
| Glimmer | 1,00 |
| Eisenoxid | 1,00 |
| Titandioxid | 2,50 |
| Talkum | 5,00 |
| EDTA | 0,50 |
| Parfüm, Konservierungsmittel, NaOH, Antioxidantien etc. | q.s. |
| Wasser | ad 100,00 |

### Beispiel 2 (Emulsions-Make-up):

| | Gew. % |
|---|---|
| Cyclomethicon | 18,00 |
| Phenyltrimethicon | 3,00 |
| PPG-3 Benzylethermyristat | 0,75 |
| Butylmethoxydibenzoylmethan | 1,50 |
| Cetyl PEG/PPG - 10/1 Dimethicon (z.B. Abil EM 90) | 4,00 |
| Paraffinöl | 3,00 |
| Eisenoxid | 2,30 |
| Titandioxid | 4,50 |
| Talkum | 2,00 |
| Natrium Chlorid | 2,00 |
| Quaternium-18 Hectorit | 0,30 |
| Propylen Carbonat | 0,08 |
| Parfüm, Konservierungsmittel, NaOH, Antioxidantien etc. | q.s. |
| Wasser | ad 100,00 |

## Patentansprüche

1. Kosmetische Zubereitung für die topische Anwendung, **dadurch gekennzeichnet, dass** sie in einem kosmetisch akzeptablen Träger
a) mindestens einen UV-Filter gewählt aus der Gruppe 4-(tert.-Butyl)-4'-methoxydibenzoylmethan (CAS-Nr. 70356-09-1) und 4-Methoxyzimtsäure-2-ethylhexylester sowie
b) PPG-3 Benzylethermyristat (CAS 403517-45-3)
enthält.

2. Kosmetische Zubereitung nach Anspruch 1, **dadurch gekennzeichnet, dass** die Zubereitung PPG-3 Benzylethermyristat (CAS 403517-45-3) in einer Konzentration von 0,1 bis 10 Gewichts-%, enthält.

3. Kosmetische Zubereitung nach einem der vorhergehenden Ansprüche, **dadurch gekennzeichnet, dass** die Zubereitung den oder die UV-Filter gewählt aus der Gruppe 4-(tert.-Butyl)-4'-methoxydibenzoylmethan (CAS-Nr. 70356-09-1) und 4-Methoxy-zimtsäure-2-ethylhexylester in einer Gesamtkonzentration von 2 bis 15 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung weitere organische UV-A- und/oder UV-B-Filter enthält.

5. Kosmetische Zubereitung nach einem der vorhergehenden Ansprüche, **dadurch gekennzeichnet, dass** der oder die weiteren organischen UV-Filter gewählt werden aus der Gruppe der Verbindungen der Anthralinate, Cinnamate, Salicylate, Derivate des Camphers, Derivate des Benzophenons, Derivate der β,β-Diphenylacrylate, Derivate von Benzotriazol, Derivate von Benzalmalonaten, Derivate von Benzimidazolen, Imidazoline, Derivate von bis-Benzoazolyl, Derivate von p-Aminobenzoesäure, Derivate von Methylen-bis-(hydroxyphenylbenztriazol), polymere Filter, Silikonfilter, Dimere Derivate von α-Alkylstyren, 4, 4-Diarylbutadien.

6. Kosmetische Zubereitung nach einem der vorhergehenden Ansprüche, **dadurch gekennzeichnet**, das einer oder mehrere organische Filter gewählt werden aus der Gruppe der folgenden Verbindungen:
- Ethylhexylsalicylat,
- Octocrylen,
- Phenylbenzimidazolsulfonsäure,
- Terephtalidendicampfersulfonsäure,
- Benzophenon-3,
- Benzophenon-4,
- Benzophenon-5,
- 4-Methylbenzylidenkampfer,
- Dinatriumphenyldibenzimidaoltetrasulfonat,
- Anisotriazin (Ethylhexyloxyphenol Methoxyphenyl Triazin),
- Ethylhexyltriazon,
- Diethylhexylbutamidotriazon,
- 2,4,6-tris-(4'-aminodiisobutylbenzalmalonat)-s-triazin,
- Methylenbis-Benzotriazolyltetramethylbutylphenol,
- Drometrizoltrisiloxan,
- 1,1-Dicarboxy(2,2'-dimethylpropyl)-4,4-diphenylbutadien.

7. Zubereitung nach einem der vorhergehenden Ansprüche, **dadurch gekennzeichnet, dass** die Zubereitung einen oder mehrere UV-Filter gewählt aus der Gruppe der Verbindungen 2,4-Bis-{[4-(2-ethyl-hexyloxy)-2-hydroxy]-phenyl}-6-(4-methoxyphenyl)-1,3,5-triazin; Dioctylbutylamidotriazon; 2,4-bis-[5-1(dimethylpropyl)benzoxazol-2-yl-(4-phenyl)-imino]-6-(2-ethylhexyl)-imino-1,3,5-triazin; 4,4',4"-(1,3,5-Triazin-2,4,6-triyltriimino)-tris-benzoesäure-tris(2-ethylhexylester); 2,4,6-Tribiphenyl-4-yl-1,3,5-triazin; Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; Ethylhexylsalicylat; Terephthalidendicamphersulfonsäure; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)-benzoesäure(2-ethylhexyl)ester; 4-(Dimethylamino)benzoesäure-amylester; 4-Methoxybenzalmalonsäuredi(2-ethylhexyl)ester; Dioctylbutylamidotriazon; 4-Methoxyzimtsäureisopentylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; 2-Ethylhexyl-2-cyano-3,3-diphenylacrylat; 3-(4-(2,2-bis Ethoxycarbonylvinyl)-phenoxy)propenyl)-methoxysiloxan/Dimethylsiloxan - Copolymer; Dimethicodiethylbenzalmalonat.

8. Zubereitung nach einem der vorhergehenden Ansprüche, **dadurch gekennzeichnet, dass** sie Pigmente und/oder Nanopigmente von beschichteten und/oder unbeschichteten Metalloxiden enthält.

9. Zubereitung nach Anspruch 8, **dadurch gekennzeichnet, dass** die Pigmente und/oder Nanopigmente gewählt werden aus den Oxiden des Titans, Zinks, Eisens, Zirkoniums, Cers, und deren Mischungen.

10. Zubereitung nach einem der vorhergehenden Ansprüche, **dadurch gekennzeichnet, dass** sie als weiteres mindestens eine Verbindung zur Bräunung der Haut enthält.

11. Zubereitung nach einem der vorhergehenden Ansprüche, **dadurch gekennzeichnet, dass** die Zubereitung als weitere Komponenten mindestens eine Verbindung gewählt aus der Gruppe der Verbindungen der Lipide, der organischen Lösungsmittel, der ionischen oder nichtionischen Verdickungsmittel, die Haut beruhigende Stoffe, Antioxidantien, Radikalfänger, trübungsmittel, Stabilisatoren, Emulgatoren, Hauterweichungsmittel, Silikone, α-Hydroxysäuren, Entschäumer, Feuchthaltemittel, Vitamine, Substanz P Antagonisten, Füllstoffe, Polymere, Treibmittel, Säuren bzw. Basen zur pH-Wert-Einstellung, Farbstoffe.

12. Zubereitung nach einem der vorhergehenden Ansprüche, **dadurch gekennzeichnet, dass** es sich um eine Emulsion, Dispersion oder eine gelförmige Zubereitung handelt.

13. Kosmetische Zubereitung nach einem der vorhergehenden Ansprüche, **dadurch gekennzeichnet, dass** die Zubereitung Tocopherylacetat enthält.

14. Kosmetische Zubereitung nach einem der vorhergehenden Ansprüche, **dadurch gekennzeichnet, dass** die Zubereitung Phenoxyethanol und/oder Parabene wie Methylparaben oder Propylparaben enthält.
